# EUROPEAN PATENT APPLICATION

(11) **EP 0 923 938 A1**
(43) Date of publication of application: **23.06.1999**
(21) Application number: 98907241.8
(22) Date of filing: 16.03.1998
(51) Int. Cl.: A61K 38/06, A61K 7/00, A61K 7/48, A61K 35/78

(54) **IMMUNOPOTENTIATORS**

(30) Priority: 21.03.1997 JP 8766097; 05.06.1997 JP 16327597; 26.06.1997 JP 18588497; 26.06.1997 JP 18588597; 06.08.1997 JP 22424097; 07.08.1997 JP 22564297; 07.08.1997 JP 22564397
(71) Applicant: SHISEIDO COMPANY LIMITED, Chuo-ku, Tokyo 104-8010 (JP)
(72) Inventor: YAGI, Eiichiro, Shiseido Company, Ltd., Yokohama-shi, Kanagawa 223-8553 (JP); NAGANUMA, Masako-Shiseido Research Center (1), Yokohama-shi, Kanagawa 223-8553 (JP); IWAI, Ichiro-Shiseido Research Center (1), Yokohama-shi, Kanagawa 223-8553 (JP); HATAO, Masato-Shiseido Research Center (1), Yokohaa-shi, Kanagawa 223-8553 (JP); YAMAGUCHI, Kenji-Shiseido Research Center (1), Yokohama-shi, Kanagawa 223-8553 (JP); WADA, Genji-Shiseido Research Center (1), Yokoham-a-shi, Kanagawa 223-8553 (JP)
(74) Representative: TER MEER STEINMEISTER & PARTNER GbR
(86) International application number: JP9801094
(87) International publication number: WO9842363

(57) **Abstract**

An immunopotentiator for preventing ultraviolet light-induced skin immunosuppression or a drug against ultraviolet light-induced skin immunosuppression which contains glutathione or Scutellaria root extract. Also, an immunopotentiator or a drug against immunosuppression which contains linden extract, clove extract, Geranium herb extract or rosemary extract. They can prevent a reduction of immune functions due to ultraviolet light.

## Description

### FIELD OF THE INVENTION

The present invention relates to a skin immunopotentiator or an endermic liniment against ultraviolet light-induced skin immunosuppression for preventing skin immunosuppresion due to exposure to ultraviolet light by external application.

### BACKGROUND OF THE INVENTION

Skin is an organ which is located in the outermost layer of a living body and it also is an organ which incurs physical, chemical and biological invasion most intensely and directly. Recently it has become clear that the skin is also the most well developed immune organ.

The skin consists of corneum cells of the epidermis, Langerhans' cells, dendritic cells of the corium, vascular endothelial cells, macrophages, etc. The Langerhans' cells are believed to play a central role in skin immune function by their ability to process and present antibodies. It is believed that they promptly contact and deal with an antigen which has entered from the outside as a foreign entity and then move to lymph glands and present the antigen to the T cells, initiating a subsequent series of immune response reactions.

Recently, the possibility of ultraviolet light promoting carcinogenesis through a reduction in the skin immune reaction due to ultraviolet light, in addition to the carcinogenic properties of ultraviolet light itself, has been pointed out. It is quite important for the purposes of preventing carcinogenesis due to ultraviolet light to protect against ultraviolet light using anti-sun exposure cosmetics such as sunscreens. However, even during seasons when sunscreens are not used on a daily basis, the immune suppression actions may take place and there is also a concern about various adverse effects other than carcinogenesis on living bodies.

It is also known that, just as the skin immune functions are reduced by aging, various other causes in addition to ultraviolet light reduce the skin immune functions.

Because of the aforementioned reasons, there has been an urgent need to develop drugs with immunopotentiating actions or anti-immunosuppression functions which can be used on a daily basis.

However, a detailed investigation of the relationships between the various forms of skin immusuppresion due to different causes has not been conducted. For example, there is no guarantee that a substance which controls the skin immunosuppression due to aging can control the skin immunosuppression due to other causes.

Also, there has not been enough research about the prevention of skin immunosuppression due to ultraviolet light compared with research about skin immunosuppression due to aging.

For example, glutathione is known to be administered orally as a substance which controls the skin immunosuppression due to aging (refer to Fragrance Journal No. 82, 1987, p65). However, there has been no research about whether or not the external application of glutathione can control the skin immunosuppression due to aging or whether or not it can control the skin immunosuppression due to ultraviolet light.

The inventors investigated various substances for their effect on preventing the immunosuppressive actions of ultraviolet light and as a result discovered that glutathione, which, when administered orally, has an effect of preventing immunosuppression due to aging, can also prevent immunosuppression due to ultraviolet light when applied externally. The present invention was completed based on this discovery.

There has been no report on externally applied glutathione regarding its immunopotentiating actions or its effects of alleviating/preventing immunosuppression when used for controlling immunosuppression due to ultraviolet light. The present invention was completed by the discovery to the effect that glutathione can very distinctively, through external application, alleviate/prevent a reduction in immune functions due to ultraviolet light, a discovery which could not have been foreseen by the present party.

For Scutellaria root extract, it has been known that Baicalein, one of its ingredients, has cell potentiating actions (refer to Japanese unexamined patent publication Tokkai Sho 64-50877). However, there has been no research on whether it can control the skin immunosuppression due to ultraviolet light.

The inventors investigated various substances for their effect on preventing the skin immunosuppressive actions of ultraviolet light and as a result discovered that Scutellaria root extract can prevent immunosuppression due to ultraviolet light. The present invention was completed based on this discovery.

There has been no report on Scutellaria root extract regarding its immunopotentiating actions or its effects of alleviating/preventing immunosuppression when used for controlling immunosuppression due to ultraviolet light. The present invention was completed by the discovery to the effect that Scutellaria root extract can very distinctively alleviate/prevent a reduction in immune functions due to ultraviolet light, a discovery which could not have been foreseen by the present party.

For linden extract, clove extract, Geranium herb extract and rosemary extract, there has been no research regarding whether they can control the reduction of the immune functions.

For the purpose of solving this problem, the inventors investigated various substances for the effect of their on preventing immunosuppressive actions and as a result discovered that linden extract, clove extract, Geranium herb extract and rosemary extract had distinctive immunopotentiating actions as well as the effects of alleviating and preventing a reduction in immune functions, thus completing the present invention.

There has been no report about the immunopotentiating actions and the alleviation/prevention of immunosuppression by linden extract, clove extract, Geranium herb extract and rosemary extract. The present invention was completed by the new discovery to the effect that linden extract has immunopotentiating actions and alleviates/prevents the reduction of the immune functions due to ultraviolet light.

### DISCLOSURE OF THE INVENTION

The present invention provides an immunopotentiator for preventing ultraviolet light-induced skin immunosuppression which characteristically contains glutathione.

Also, the present invention provides a drug against ultraviolet light-induced skin immunosuppression which characteristically contains glutathione.

Furthermore, the present invention provides an immunopotentiating endermic liniment for preventing ultraviolet light-induced skin immunosuppression.

Also, the present invention provides an endermic liniment against ultraviolet light-induced skin immunosuppression which characteristically contains glutathione.

Furthermore, the present invention provides an immunopotentiator for preventing ultraviolet light-induced skin immunosuppression which characteristically contains Scutellaria root extract.

Also, the present invention provides a drug against ultraviolet light-induced skin immunosuppression which characteristically contains Scutellaria root extract.

Furthermore, the present invention provides an immunopotentiator which characteristically contains linden extract.

Also, the present invention provides a drug against immunosuppression which characteristically contains linden extract.

Furthermore, the present invention provides an immunopotentiator which characteristically contains clove extract.

Also, the present invention provides a drug against immunosuppression which characteristically contains clove extract.

Furthermore, the present invention provides an immunopotentiator which characteristically contains Geranium herb extract.

Also, the present invention provides a drug against immunosuppression which characteristically contains Geranium herb extract.

Furthermore, the present invention provides an immunopotentiator which characteristically contains rosemary extract.

Also, the present invention provides a drug against immunosuppression which characteristically contains rosemary extract.

### BRIEF EXPLANATION OF THE DRAWING

FIG. 1 shows the reduction in antigen presentation by Langerhans' cells due to UV exposure and the effect of glutathione (GSH) to prevent this reduction.
FIG. 2 shows the suppression of expression of the intercellular adhesive molecules-1 (ICAM-1) in Langerhans' cells due to UV irradiation and the preventive effect of Scutellaria root extract.
FIG. 3 shows the suppression of expression of the intercellular adhesive molecules-1 (ICAM-1) in Langerhans' cells due to UV irradiation and the preventive effect of linden extract.
FIG. 4 shows the suppression of expression of the intercellular adhesive molecules-1 (ICAM-1) in Langerhans' cells due to UV irradiation and the preventive effect of clove extract.
FIG. 5 shows the suppression of expression of the intercellular adhesive molecules-1 (ICAM-1) in Langerhans' cells due to UV irradiation and the preventive effect of Geranium herb extract.
FIG. 6 shows the suppression of expression of the intercellular adhesive molecules-1 (ICAM-1) in Langerhans' cells due to UV irradiation and the preventive effect of rosemary extract.

### THE BEST MODES OF THE EMBODIMENTS

The configuration of the present invention is described below.

Glutathione, which is used in the skin immunopotentiator or the drug against skin immunosuppression of the present invention, is a SH compound which exists most abundantly in a living body. It enzymatically and/or non-enzymatically reacts with the disulfides of proteins and such and has a function of maintaining the SH's. This reaction converts it to the oxidized form of glutathione.

Scutellaria root extract, which is used in the skin immunopotentiator or the drug against skin immunosuppression of the present invention, is an organic solvent extract of the root of Scutellaria baicalensis Georgi, a plant of the Lamiaceae family. For example, dried Scutellaria root powder or non-dried cut-up Scutellaria root is stirred in water or alcohol such as methanol, ethanol, propylene glycol and 1,3-butylene glycol for extraction while being heated up to 30-70 °C for 1-10 hours or at room temperature for 1-20 days. After filtration, the filtrate is concentrated and then this concentrate is stirred with purified water to precipitate yellow powder, which is dried for use. In the present invention, the Scutellaria root extract can be used at the concentrate stage or at the dry stage.

Linden extract, which is used in the immunopotentiator or the drug against immunosuppression of the present invention, is a water or organic solvent extract of linden, which is a plant of the Tilia family (Tilia platyphyllos Scop., Tilia cordata Mill. and Tilia europaea). For example, dried linden powder or non-dried cut-up linden is stirred in water or alcohol such as methanol, ethanol, propylene glycol and 1,3-butylene glycol for extraction while being heated up to 30-70°C for 1-10 hours or at room temperature for 1-20 days. After filtration, the filtrate is concentrated and then dried by vacuum concentration. In the present invention, the linden extract can be used at the concentrate stage or at the dried solid stage.

Clove extract, which is used in the immunopotentiator or the drug against immunosuppression of the present invention, is a water or organic solvent extract of clove (Syzygium aromaticum Merrill et Perry) of the Myrtaceae family. For example, dried powder of buds, leaves, seeds, the above-ground plant or the whole plant of clove or non-dried sliced clove buds is stirred in water, methanol, ethanol, propylene glycol, 1,3-butylene glycol, butanol, chloroform, dichloromethane, carbon tetrachloride, ethyl acetate, ether, etc. or a mixed solution of these for extraction while being heated up to 30-70°C for 1-10 hours or at room temperature for 1-20 days. After filtration, the filtrate is concentrated and then dried by vacuum concentration. In the present invention, the clove extract can be used at the concentrate stage or at the dried solid stage.

Geranium herb extract, which is used in the immunopotentiator or the drug against immunosuppression of the present invention, is a water or organic solvent extract of Geranium thunbergii of the Geraniaceae family. For example, dried powder of the above-ground plant, flowers, seeds, fruits, leaves or the whole plant of Geranium thunbergii or non-dried sliced Geranium thunbergii is stirred in water, methanol, ethanol, propylene glycol, 1,3-butylene glycol, butanol, chloroform, dichloromethane, carbon tetrachloride, ethyl acetate, ether, etc. or a mixed solution of these for extraction while being heated up to 30-70°C for 1-10 hours or at room temperature for 1-20 days. After filtration, the filtrate is concentrated and then dried by vacuum concentration. In the present invention, the Geranium herb extract can be used at the concentrate stage or at the dried solid stage.

The blend ratio of glutathione in the immunopotentiator or the drug against immunosuppression of the present invention, as a dry weight, is 0.005-20.0 wt%, more preferably 0.01-10.0 wt%, of the total immunopotentiator or the drug against immunosuppression. A blend ratio less than 0.005 wt% would not be preferable because then the effect of the immunopotentiator or the drug against immunosuppression of the present invention would not be sufficiently exhibited. A blend ratio more than 20 wt% would not be preferable either because then pharmaceutical preparation would become difficult. No significant increase in the effect is observed when 10.0 wt% or more is blended.

The blend ratio of Scutellaria root extract, linden extract, clove extract, Geranium herb extract or rosemary extract in the immunopotentiator or the drug against immunosuppression of the present invention, as a dry weight, is 0.0005-10.0 wt%, more preferably 0.001-5.0 wt%, of the total immunopotentiator or the drug against immunosuppression. A blend ratio less than 0.0005 wt% would not be preferable because then the effect of the immunopotentiator or the drug against immunosuppression of the present invention would not be sufficiently exhibited. A blend ratio more than 10 wt% would not be preferable either because then pharmaceutical preparation would become difficult. No significant increase in the effect is observed when 5.0 wt% or more is blended.

In addition to the essential ingredient described above, the skin immunopotentiator or the drug against skin immunosuppression of the present invention can contain, as necessary, those ingredients such as are normally used in cosmetics, drugs, etc., in the form of an endermic liniment, including whitening agents, humectants, antioxidants, oil-based ingredients, ultraviolet light absorbents, anti-inflammatory agents, surfactants, thickeners, alcohols, powdered ingredients, colorings, water-based ingredients, water and various skin nutrients.

The skin immunopotentiator or the drug against skin immunosuppression of the present invention can be in any form which is conventionally used as an endermic liniment, including ointment, cream, emulsion, lotion, facial packs and bath additives. The skin immunopotentiator or the drug againstskin immunosuppression of the present invention is highly useful as an immunopotentiating cosmetic or a cosmetic against skin immunosuppression.

### EXAMPLES

The present invention is described in detail below by referring to examples. The present invention is not limited to these examples. The blend ratios are in weight percent units.

### [1] Examples for the inventions of claims 1-4

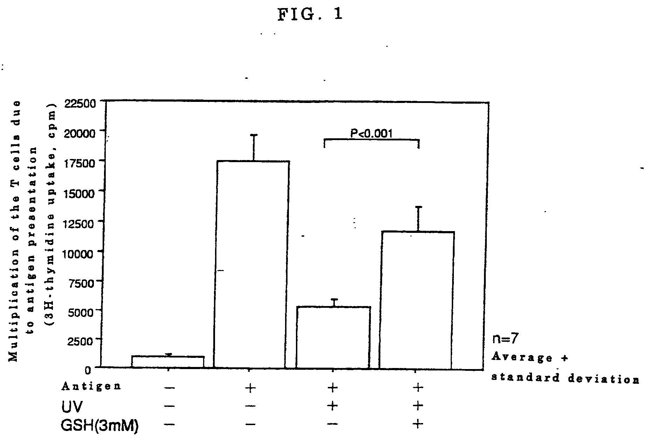

An antigen (trinitrobenzenesulfonic acid, 1 mg/ml) was added to Langerhans' cells and, after cleaning, they were mixed and cultured with T cells obtained by purifying lymph gland cell floating fluid with a nylon fiber column (Wako). As a result, the Langerhans' cells presented the antigen to the T cells and the T cells multiplied. However, when the antigen was added after irradiating the Langerhans' cells with UV and then the mixed culture with the T cells was conducted, a reduction in the multiplication of the T cells was observed because the antigen presentation function of the Langerhans' cells was suppressed. We added 3 mM of glutathione (GSH) during the ultraviolet light irradiation to study the protection effect of glutathione against the suppression of the antigen presentation function of the Langerhans' cells by UV. The results are shown in FIG. 1. The vertical axis of FIG. 1 shows the multiplication of the T cells. An increase in T cells indicates that the immune functions are working. The horizontal axis shows whether the addition of the antigen, the UV irradiation and the addition of glutathione (GSH) were carried out or not by using "+" or "-" ("+" indictates the irradiation or the addition was carried out and "-" indicates the irradiation or the addition did not take place). FIG. 1 indicates that the T cells multiply (17,500) when only the antigen was added but the number of the T cells decreases (5,000) when they were irradiated with ultraviolet light. When glutathione was added, the multiplication of the T cells recovered (11,000). Therefore, it was verified that glutathione has superior immunopotentiating actions and effects of alleviating immunosuppression.

Examples of using glutathione as a skin immunopotentiator or a drug against skin immunosuppression for the purpose of externally applying it to prevent a reduction in the immune functions due to ultraviolet light are described below.

### "Example 1 Cream"

| (Recipe) | |
|---|---|
| Stearic acid | 5.0 wt% |
| Stearyl alcohol | 4.0 |
| Isopropylmyristate | 18.0 |
| Glycerine monostearic ester | 3.0 |
| Propylene glycol | 10.0 |
| Glutathione | 0.01 |
| Paraaminobenzoic acid | 0.5 |
| Caustic potash | 0.2 |
| Preservative | Appropriate amount |
| Perfume | Appropriate amount |
| Ion exchanged water | Balance |

### (Preparation method)

Propylene glycol, glutathione and caustic potash were added to ion exchanged water and dissolved, then heated up to and maintained at 70 °C (water phase). The other ingredients were mixed and heat-melted and then the temperature was maintained at 70 °C (oil phase). The oil phase was gradually added to the water phase and, after all was added, the temperature was maintained at the same temperature to allow the reaction to occur. Finally, the product was homogeneously emulsified using a homogenizer and cooled to 30 °C while being thoroughly stirred.

### "Example 2 Cream"

| (Recipe) | |
|---|---|
| Stearic acid | 2.0 wt% |
| Stearyl alcohol | 7.0 |
| Lanolin hydrate | 2.0 |
| Squalane | 5.0 |
| 2-octyldodecyl alcohol | 6.0 |
| Polyoxyethylene (25 moles) cetyl alcohol ether | 3.0 |
| Glycerine monostearic ester | 2.0 |
| Propylene glycol | 5.0 |
| Glutathione | 0.05 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and, after pre-emulsification, the product was homogeneously emulsified using a homogenizer and cooled to 30 °C while being thoroughly stirred.

### "Example 3 Cream"

| (Recipe) | |
|---|---|
| Solid paraffin | 5.0 wt% |
| Beeswax | 10.0 |
| Vaseline | 15.0 |
| Liquid paraffin | 41.0 |
| Glycerine monostearic ester | 2.0 |
| Polyoxyethylene (20 moles) sorbitan monolauric ester | 2.0 |
| Soap powder | 0.1 |
| Borax | 0.2 |
| Glutathione | 0.05 |
| Ascorbic acid | 2.0 |
| Ethyl paraben | 0.3 |
| Perfume | Appropriate amount |
| Ion exchanged water | Balance |

### (Preparation method)

Soap powder and borax were added to ion exchanged water and dissolved, heated up to and maintained at 70 °C (water phase). The other ingredients were mixed and heat-melted and then the temperature was maintained at 70 °C (oil phase). The oil phase was gradually added to the water phase while being stirred to initiate the reaction. After the completion of the reaction, the product was homogeneously emulsified using a homogenizer and cooled to 30 °C while being thoroughly stirred.

### "Example 4 Emulsion"

| (Recipe) | |
|---|---|
| Stearic acid | 2.5 wt% |
| Cetyl alcohol | 1.5 |
| Vaseline | 5.0 |
| Liquid paraffin | 10.0 |
| Polyoxyethylene (10 moles) monooleic ester | 2.0 |
| Polyethylene glycol 1500 | 3.0 |
| Triethanolamine | 1.0 |
| Carboxyvinyl polymer (product name: Carbopol 941 from B. F. Goodrich Chemical company) | 0.05 |
| Glutathione | 0.01 |
| Octyl paradimethylaminobenzoate | 1.0 |
| Sodium hydrogensulfite | 0.01 |
| Arbutin | 3.5 |
| Ethyl paraben | 0.3 |
| Perfume | Appropriate amount |
| Ion exchanged water | Balance |

### (Preparation method)

The carboxyvinyl polymer was dissolved in a small amount of ion exhanged water (A phase). Polyethylene glycol 1500 and triethanolamine were added to the rest of the ion exchanged water and heated and dissolved, after which the temperature was maintained at 70 °C (water phase). The other ingredients were mixed and heat-melted and then the temperature was maintained at 70 °C (oil phase). The oil phase was added to the water phase and, after pre-emulsification, the A phase was added. The product was then homogeneously emulsified using a homogenizer and cooled to 30 °C while being thoroughly stirred.

### "Example 5 Emulsion"

| (Recipe) | |
|---|---|
| Microcrystalline wax | 1.0 wt% |
| Beeswax | 2.0 |
| Lanolin | 20.0 |
| Liquid paraffin | 10.0 |
| Octyl paramethylaminobenzoate | 3.0 |
| Squalane | 5.0 |
| Sorbitan sesquioleic ester | 4.0 |
| Polyoxyethylene (20 moles) sorbitan monooleic ester | 1.0 |
| Propylene glycol | 7.0 |
| Glutathione | 10.0 |
| Magnesium ascrobate phosphate | 3.0 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and homogeneously emulsified using a homogenizer. After emulsification, the product was cooled to 30 °C while being thoroughly stirred.

### "Example 6 Jelly"

| (Recipe) | |
|---|---|
| 95% ethyl alcohol | 10.0 wt% |
| Dipropylene glycol | 15.0 |
| Polyoxyethylene (50 moles) oleyl alcohol ether | 2.0 |
| Carboxyvinyl polymer (product name: Carbopol 940 from B. F. Goodrich Chemical company) | 1.0 |
| Caustic soda | 0.15 |
| L-arginine | 0.1 |
| Isopropyl paramethoxycinnamate | 0.1 |
| Titanium oxide | 5.0 |
| Glutathione | 7.0 |
| Sodium 2-hydroxy-4-methoxybenzophenonesulfonate | 0.05 |
| Ethylenediamine-tetraacetic acid trisodium dihydrate | 0.05 |
| Methyl paraben | 0.2 |
| Perfume | Appropriate amount |
| Ion exchanged water | Balance |

### (Preparation method)

Carbopol 940 was homogeneously dissolved in ion exhanged water. Glutathione and polyoxyethylene (50 moles) oleyl alcohol ether were dissolved in 95% ethanol and added to the water phase. The other ingredients were added and the mixture was neutralized and thickened by caustic soda and/or L-arginine.

### "Example 7 Essence"

| (Recipe) (A phase) | |
|---|---|
| Ethyl alcohol (95%) | 10.0 wt% |
| Polyoxyethylene (20 moles) octyldodecanol | 1.0 |
| Pantothenyl ethyl ether | 0.1 |
| Glutathione | 1.5 |
| Methyl paraben | 0.15 |

| (B phase) | |
|---|---|
| Potassium hydroxide | 0.1 |

| (C phase) | |
|---|---|
| Glycerine | 5.0 |
| Dipropylene glycol | 10.0 |
| Carboxyvinyl polymer (product name: Carbopol 940 from B. F. Goodrich Chemical company) | 0.2 |
| Purified water | Balance |

### (Preparation method)

The A phase and C phase were, separately, each homogeneously dissolved. The A phase was then added to the C phase and solubilized. Finally, the B phase was added and a container was filled with the product.

### "Example 8 Facial pack"

| (Recipe) (A phase) | |
|---|---|
| Dipropylene glycol | 5.0 wt% |
| Polyoxyethylene (60 moles hydrogenated castor oil | 5.0 |

| (B phase) | |
|---|---|
| Glutathione | 0.01 |
| Olive oil | 5.0 |
| Tocopherol acetate | 0.2 |
| Ethyl paraben | 0.2 |
| Perfume | 0.2 |

| (C phase) | |
|---|---|
| Polyvinyl alcohol (degree of saponification: 90, degree of polymerization: 2,000) | 13.0 |
| Ethanol | 7.0 |
| Purified water | Balance |

### (Preparation method)

Each of the A phase, B phase and C phase was homogeneously dissolved. The B phase was added to the A phase and solubilized. The C phase was then added and a container was filled with the product.

### "Example 9 Solid foundation"

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octanoate | 2.0 |
| Glutathione | 1.0 |
| Preservative | Appropriate amount |
| Perfume | Appropriate amount |

### (Preparation method)

The powder ingredients, from talc to black iron oxide as listed above, were thoroughly mixed with a blender. The oil ingredients, from squalane to isocetyl octanoate as listed above, as well as glutathione, the preservative and the perfume, were added and, after thorough kneading, a container was filled with the product.

### "Example 10 Emulsified foundation (cream type)"

| (Recipe) (Powder portion) | |
|---|---|
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene modified dimethyl polysiloxane | 4.0 |

| (Water phase) | |
|---|---|
| Purified water | 50.0 |
| 1,3-butylene glycol | 4.5 |
| Glutathione | 1.5 |
| Ascorbyl glucoside | 1.0 |
| Sorbitan sesquioleic ester | 3.0 |
| Preservative | Appropriate amount |
| Perfume | Appropriate amount |

### (Preparation method)

After heating and stirring the water phase, the powder portion was added to it and the mixture was treated with a homogenizer. The oil phase, healed and mixed, was then added and the resulting mixture was treated with a homogenizer. The perfume was then added while stirring and the product was cooled down to room temperature.

### [2] Examples for the inventions of claims 5-6

The immunopotentiating action and the effect of alleviating/preventing ultraviolet light-induced immunosuppression of Scutellaria root extract were investigated by observing the prevention against the suppression of expression of the intercellular adhesive molecules-1 (ICAM-1) in Langerhans' cells due to UV irradiation.

### "Scutellaria root extract"

The Scutellaria root extract used in the following examples was prepared by adding water to thinly sliced skinned root of Scutellaria baicalensis Georgi, raising the temperature up to 50 °C, adding ethanol, carrying out extraction for five hours, filtering and removing the solvent from the filtrate to obtain a concentrate.

### [Testing methods and results: Prevention against the suppression of expression of the intercellular adhesive molecules-1 (ICAM-1) in Langerhans' cells due to UV irradiation.]

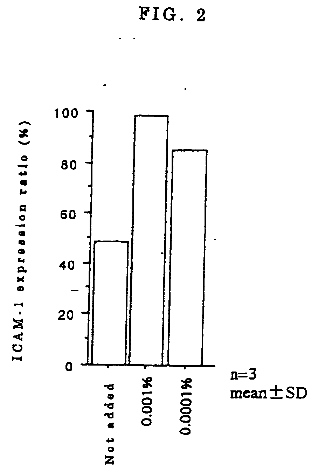

Langerhans' cells prepared by treating human skin epidermis with 0.5% trypsin was irradiated with UVA (5 J/cm², BLB lamp) and then cultured in a CO₂ incubator, with RPMI1640/10%FBS, for 24 hours at 37 °C . After the culture process, the cells were treated with the anti-MHC class II antibody labeled with FITC (from PharMingen) and the anti-ICAM-1 antibody labeled with PE (from PharMingen). A flow cytometer (XL from Epix) was used to analyse 3 x 10⁴ of the cells to measure the ICAM-1 expression. The result is shown in FIG. 2. The vertical axis of FIG. 2 shows the ICAM-1 expression ratio (%) and the horizontal axis shows the presence or absence of the Scutellaria root extract (final concentration in wt% unit). FIG. 2 indicates that the suppression of expression of the intercellular adhesive molecules-1 (ICAM-1) in Langerhans' cells due to UV irradiation is prevented by the addition of the Scutellaria root extract.

Examples of using Scutellaria root extract as an immunopotentiator or a drug against immunosuppression are described below.

### "Example 1 Cream"

| (Recipe) | |
|---|---|
| Stearic acid | 5.0 wt% |
| Stearyl alcohol | 4.0 |
| Isopropylmyristate | 18.0 |
| Glycerine monostearic ester | 3.0 |
| Propylene glycol | 10.0 |
| Scutellaria root extract | 0.01 |
| Paraaminobenzoic acid | 0.5 |
| Caustic potash | 0.2 |
| 2-ethylhexylparamethoxycinnamic acid | 3.0 |
| Preservative | Appropriate amount |
| Perfume | Appropriate amount |
| Ion exchanged water | Balance |

### (Preparation method)

Propylene glycol, Scutellaria root extract and caustic potash were added to ion exchanged water and dissolved, then heated up to and maintained at 70 °C (water phase). The other ingredients were mixed and heat-melted and then the temperature was maintained at 70 °C (oil phase). The oil phase was gradually added to the water phase and, after all was added, the temperature was maintained at the same temperature to allow the reaction to occur. Finally, the product was homogeneously emulsified using a homogenizer and cooled to 30 °C while being thoroughly stirred.

### "Example 2 Cream"

| (Recipe) | |
|---|---|
| Stearic acid | 2.0 wt% |
| Stearyl alcohol | 7.0 |
| Lanolin hydrate | 2.0 |
| Squalane | 5.0 |
| 2-octyldodecyl alcohol | 6.0 |
| Polyoxyethylene (25 moles) cetyl alcohol ether | 3.0 |
| Glycerine monostearic ester | 2.0 |
| Propylene glycol | 5.0 |
| 2-ethylhexylparamethoxycinnamic acid | 10.0 |
| Scutellaria root extract | 0.05 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and, after pre-emulsification, the product was homogeneously emulsified using a homogenizer and cooled to 30 °C while being thoroughly stirred.

### "Example 3 Cream"

| (Recipe) | |
|---|---|
| Solid paraffin | 5.0 wt% |
| Beeswax | 10.0 |
| Vaseline | 15.0 |
| Liquid paraffin | 41.0 |
| Glycerine monostearic ester | 2.0 |
| Polyoxyethylene (20 moles) sorbitan monolauric ester | 2.0 |
| Soap powder | 0.1 |
| 2-ethylhexylparamethoxycinnamic acid | 3.0 |
| Borax | 0.2 |
| Scutellaria root extract | 0.05 |
| Ascorbic acid | 2.0 |
| Ethyl paraben | 0.3 |
| Perfume | Appropriate amount |
| Ion exchanged water | Balance |

### (Preparation method)

Soap powder and borax were added to ion exchanged water and dissolved, heated up to and maintained at 70 °C (water phase). The other ingredients were mixed and heat-melted and then the temperature was maintained at 70 °C (oil phase). The oil phase was gradually added to the water phase while being stirred to initiate the reaction. After the completion of the reaction, the product was homogeneously emulsified using a homogenizer and cooled to 30 °C while being thoroughly stirred.

### "Example 4 Emulsion"

| (Recipe) | |
|---|---|
| Stearic acid | 2.5 wt% |
| Cetyl alcohol | 1.5 |
| Vaseline | 5.0 |
| Liquid paraffin | 10.0 |
| Polyoxyethylene (10 moles) monooleic ester | 2.0 |
| Polyethylene glycol 1500 | 3.0 |
| Triethanolamine | 1.0 |
| Carboxyvinyl polymer (product name: Carbopol 941 from B. F. Goodrich Chemical company) | 0.05 |
| Scutellaria root extract | 0.01 |
| Octyl paradimethylaminobenzoate | 1.0 |
| Sodium hydrogensulfite | 0.01 |
| Arbutin | 3.5 |
| Ethyl paraben | 0.3 |
| Perfume | Appropriate amount |
| Ion exchanged water | Balance |

### (Preparation method)

The carboxyvinyl polymer was dissolved in a small amount of ion exhanged water (A phase). Polyethylene glycol 1500 and triethanolamine were added to the rest of the ion exchanged water and heated and dissolved, after which the temperature was maintained at 70 °C (water phase). The other ingredients were mixed and heat-melted and then the temperature was maintained at 70 °C (oil phase). The oil phase was added to the water phase and, after pre-emulsification, the A phase was added. The product was then homogeneously emulsified using a homogenizer and cooled to 30 °C while being thoroughly stirred.

### "Example 5 Emulsion"

| (Recipe) | |
|---|---|
| Microcrystalline wax | 1.0 wt% |
| Beeswax | 2.0 |
| Lanolin | 20.0 |
| Liquid paraffin | 10.0 |
| Octyl paramethylaminobenzoate | 3.0 |
| 2-ethylhexylparamethoxycinnamic acid | 4.0 |
| Squalane | 5.0 |
| Sorbitan sesquioleic ester | 4.0 |
| Polyoxyethylene (20 moles) sorbitan monooleic ester | 1.0 |
| Propylene glycol | 7.0 |
| Scutellaria root extract | 10.0 |
| Magnesium ascrobate phosphate | 3.0 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and homogeneously emulsified using a homogenizer. After emulsification, the product was cooled to 30 °C while being thoroughly stirred.

### "Example 6 Jelly"

| (Recipe) | |
|---|---|
| 95% ethyl alcohol | 10.0 wt% |
| Dipropylene glycol | 15.0 |
| Polyoxyethylene (50 moles) oleyl alcohol ether | 2.0 |
| Carboxyvinyl polymer (product name: Carbopol 940 from B. F. Goodrich Chemical company) | 1.0 |
| Caustic soda | 0.15 |
| L-arginine | 0.1 |
| Isopropyl paramethoxycinnamate | 0.1 |
| Titanium oxide | 5.0 |
| Scutellaria root extract | 7.0 |
| Sodium 2-hydroxy-4-methoxybenzophenonesulfonate | 0.05 |
| Ethylenediamine-tetraacetic acid trisodium dihydrate | 0.05 |
| Methyl paraben | 0.2 |
| Perfume | Appropriate amount |
| Ion exchanged water | Balance |

### (Preparation method)

Carbopol 940 was homogeneously dissolved in ion exhanged water. Scutellaria root extract and polyoxyethylene (50 moles) oleyl alcohol ether were dissolved in 95% ethanol and added to the water phase.

The other ingredients were added and the mixture was neutralized and thickened by caustic soda and/or L-arginine.

### "Example 7 Essence"

| (Recipe) (A phase) | |
|---|---|
| Ethyl alcohol (95%) | 10.0 wt% |
| Polyoxyethylene (20 moles) octyldodecanol | 1.0 |
| Pantothenyl ethyl ether | 0.1 |
| Scutellaria root extract | 1.5 |
| Methyl paraben | 0.15 |

| (B phase) | |
|---|---|
| Potassium hydroxide | 0.1 |

| (C phase) | |
|---|---|
| Glycerine | 5.0 |
| Dipropylene glycol | 10.0 |
| Carboxyvinyl polymer (product name: Carbopol 940 from B. F. Goodrich Chemical company) | 0.2 |
| Purified water | Balance |

### (Preparation method)

The A phase and C phase were, separately, each homogeneously dissolved. The A phase was then added to the C phase and solubilized. Finally, the B phase was added and a container was filled with the product.

### "Example 8 Facial pack"

| (Recipe) (A phase) | |
|---|---|
| Dipropylene glycol | 5.0 wt% |
| Polyoxyethylene (60 moles) hydrogenated castor oil | 5.0 |

| (B phase) | |
|---|---|
| Scutellaria root extract | 0.01 |
| Olive oil | 5.0 |
| Tocopherol acetate | 0.2 |
| Ethyl paraben | 0.2 |
| Perfume | 0.2 |

| (C phase) | |
|---|---|
| Polyvinyl alcohol (degree of saponification: 90, degree of polymerization: 2,000) | 13.0 |
| Ethanol | 7.0 |
| Purified water | Balance |

### (Preparation method)

Each of the A phase, B phase and C phase was homogeneously dissolved. The B phase was added to the A phase and solubilized. The C phase was then added and a container was filled with the product.

### "Example 9 Solid foundation"

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octanoate | 2.0 |
| Scutellaria root extract | 1.0 |
| Preservative | Appropriate amount |
| Perfume | Appropriate amount |

### (Preparation method)

The powder ingredients, from talc to black iron oxide as listed above, were thoroughly mixed with a blender. The oil ingredients, from squalane to isocetyl octanoate as listed above, as well as Scutellaria root extract, the preservative and the perfume, were added and, after thorough kneading, a container was filled with the product.

### "Example 10 Emulsified foundation (cream type)"

| (Recipe) (Powder portion) | |
|---|---|
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene modified dimethyl polysiloxane | 4.0 |

| (Water phase) | |
|---|---|
| Purified water | 50.0 |
| 1,3-butylene glycol | 4.5 |
| Scutellaria root extract | 1.5 |
| Ascorbyl glucoside | 1.0 |
| Sorbitan sesquioleic ester | 3.0 |
| Preservative | Appropriate amount |
| Perfume | Appropriate amount |

### (Preparation method)

After heating and stirring the water phase, the powder portion was added to it and the mixture was treated with a homogenizer. The oil phase, heated and mixed, was then added and the resulting mixture was treated with a homogenizer. The perfume was then added while stirring and the product was cooled down to room temperature.

### [3] Examples for the inventions of claims 7-8

The immunopotentiating action and the effect of alleviating/preventing ultraviolet light-induced immunosuppression of linden extract were investigated by observing the prevention against the suppression of expression of the intercellular adhesive molecules-1 (ICAM-1) in Langerhans' cells due to UV irradiation.

### "Linden extract"

The linden extract used in the following examples was prepared by five-hour extraction of thinly sliced flowers and leaves of Tilia cordata mill. in 50% ethanol at 50 °C , followed by filtering and removal of the solvent irom the filtrate to obtain a concentrate.

### [Testing methods and results: Prevention against the suppression of expression of the intercellular adhesive molecules-1 (ICAM-1) in Langerhans' cells due to UV irradiation.]

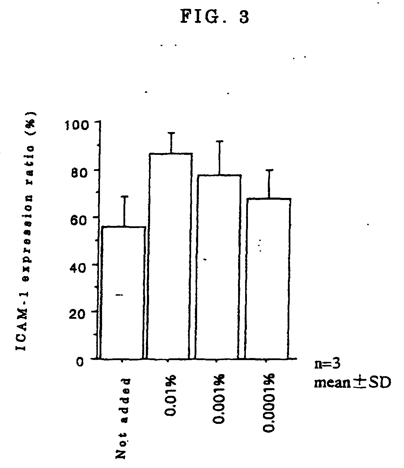

Langerhans' cells prepared by treating human skin epidermis with 0.5% trypsin was irradiated with UVA (5 J/cm², BLB lamp) and then cultured in a CO₂ incubator, with RPMI1640/10%FBS, for 24 hours at 37 °C . After the culture process, the cells were treated with the anti-MHC class II antibody labeled with FITC (from PharMingen) and the anti-ICAM-1 antibody labeled with PE (from PharMingen). A flow cytometer (XL from Epix) was used to analyse 3 x 10⁴ of the cells to measure the ICAM-1 expression. The result is shown in FIG. 3. The vertical axis of FIG. 3 shows the ICAM-1 expression ratio (%) and the horizontal axis shows the presence or absence of the linden extract (final concentration in wt% unit). FIG. 3 indicates that the suppression of expression of the intercellular adhesive molecules-1 (ICAM-1) in Langerhans' cells due to UV irradiation is prevented by the addition of the linden extract.

Examples of using linden extract as an immunopotentiator or a drug against immunosuppression are described below.

### "Example 1 Cream"

| (Recipe) | |
|---|---|
| Stearic acid | 5.0 wt% |
| Stearyl alcohol | 4.0 |
| Isopropylmyristate | 18.0 |
| Glycerine monostearic ester | 3.0 |
| Propylene glycol | 10.0 |
| Linden extract | 0.01 |
| Paraaminobenzoic acid | 0.5 |
| 2-ethylhexylparamethoxycinnamic acid | 5.0 |
| Caustic potash | 0.2 |
| Preservative | Appropriate amount |
| Perfume | Appropriate amount |
| Ion exchanged water | Balance |

### (Preparation method)

Propylene glycol, linden extract and caustic potash were added to ion exchanged water and dissolved, then heated up to and maintained at 70 °C (water phase). The other ingredients were mixed and heat-melted and then the temperature was maintained at 70 °C (oil phase). The oil phase was gradually added to the water phase and, after all was added, the temperature was maintained at the same temperature to allow the reaction to occur. Finally, the product was homogeneously emulsified using a homogenizer and cooled to 30 °C while being thoroughly stirred.

### "Example 2 Cream"

| (Recipe) | |
|---|---|
| Stearic acid | 2.0 wt% |
| Stearyl alcohol | 7.0 |
| Lanolin hydrate | 2.0 |
| Squalane | 5.0 |
| 2-octyldodecyl alcohol | 6.0 |
| Polyoxyethylene (25 moles) cetyl alcohol ether | 3.0 |
| Glycerine monostearic ester | 2.0 |
| Propylene glycol | 5.0 |
| 2-ethylhexylparamethoxycinnamic acid | 10.0 |
| Linden extract | 0.05 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and, after pre-emulsification, the product was homogeneously emulsified using a homogenizer and cooled to 30 °C while being thoroughly stirred.

### "Example 3 Cream"

| (Recipe) | |
|---|---|
| Solid paraffin | 5.0 wt% |
| Beeswax | 10.0 |
| Vaseline | 15.0 |
| Liquid paraffin | 41.0 |
| Glycerine monostearic ester | 2.0 |
| Polyoxyethylene (20 moles) sorbitan monolauric ester | 2.0 |
| Soap powder | 0.1 |
| 2-ethylhexylparamethoxycinnamic acid | 1.0 |
| Borax | 0.2 |
| Linden extract | 0.05 |
| Ascorbic acid | 2.0 |
| Ethyl paraben | 0.3 |
| Perfume | Appropriate amount |
| Ion exchanged water | Balance |

### (Preparation method)

Soap powder and borax were added to ion exchanged water and dissolved, heated up to and maintained at 70 °C (water phase). The other ingredients were mixed and heat-melted and then the temperature was maintained at 70 °C (oil phase). The oil phase was gradually added to the water phase while being stirred to initiate the reaction. After the completion of the reaction, the product was homogeneously emulsified using a homogenizer and cooled to 30 °C while being thoroughly stirred.

### "Example 4 Emulsion"

| (Recipe) | |
|---|---|
| Stearic acid | 2.5 wt% |
| Cetyl alcohol | 1.5 |
| Vaseline | 5.0 |
| Liquid paraffin | 10.0 |
| Polyoxyethylene (10 moles) monooleic ester | 2.0 |
| Polyethylene glycol 1500 | 3.0 |
| Triethanolamine | 1.0 |
| Carboxyvinyl polymer (product name: Carbopol 941 from B. F. Goodrich Chemical company) | 0.05 |
| Linden extract | 0.01 |
| Octyl paradimethylaminobenzoate | 1.0 |
| Sodium hydrogensulfite | 0.01 |
| Arbutin | 3.5 |
| Ethyl paraben | 0.3 |
| Perfume | Appropriate amount |
| Ion exchanged water | Balance |

### (Preparation method)

The carboxyvinyl polymer was dissolved in a small amount of ion exhanged water (A phase). Polyethylene glycol 1500 and triethanolamine were added to the rest of the ion exchanged water and heated and dissolved, after which the temperature was maintained at 70 °C (water phase). The other ingredients were mixed and heat-melted and then the temperature was maintained at 70 °C (oil phase). The oil phase was added to the water phase and, after pre-emulsification, the A phase was added. The product was then homogeneously emulsified using a homogenizer and cooled to 30 °C while being thoroughly stirred.

### "Example 5 Emulsion"

| (Recipe) | |
|---|---|
| Microcrystalline wax | 1.0 wt% |
| Beeswax | 2.0 |
| Lanolin | 20.0 |
| Liquid paraffin | 10.0 |
| Octyl paramethylaminobenzoate | 3.0 |
| 2-ethylhexylparamethoxycinnamic acid | 5.0 |
| Squalane | 5.0 |
| Sorbitan sesquioleic ester | 4.0 |
| Polyoxyethylene (20 moles) sorbitan monooleic ester | 1.0 |
| Propylene glycol | 7.0 |
| Linden extract | 10.0 |
| Magnesium ascrobate phosphate | 3.0 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and homogeneously emulsified using a homogenizer. After emulsification, the product was cooled to 30 °C while being thoroughly stirred.

### "Example 6 Jelly"

| (Recipe) | |
|---|---|
| 95% ethyl alcohol | 10.0 wt% |
| Dipropylene glycol | 15.0 |
| Polyoxyethylene (50 moles) oleyl alcohol ether | 2.0 |
| Carboxyvinyl polymer (product name: Carbopol 940 from B. F. Goodrich Chemical company) | 1.0 |
| Caustic soda | 0.15 |
| L-arginine | 0.1 |
| Isopropyl paramethoxycinnamate | 0.1 |
| 2-ethylhexylparamethoxycinnamic acid | 0.5 |
| Titanium oxide | 5.0 |
| Linden extract | 7.0 |
| Sodium 2-hydroxy-4-methoxybenzophenonesulfonate | 0.05 |
| Ethylenediamine-tetraacetic acid trisodium dihydrate | 0.05 |
| Methyl paraben | 0.2 |
| Perfume | Appropriate amount |
| Ion exchanged water | Balance |

### (Preparation method)

Carbopol 940 was homogeneously dissolved in ion exhanged water. Linden extract and polyoxyethylene (50 moles) oleyl alcohol ether were dissolved in 95% ethanol and added to the water phase. The other ingredients were added and the mixture was neutralized and thickened by caustic soda and/or L-arginine.

### "Example 7 Essence"

| (Recipe) (A phase) | |
|---|---|
| Ethyl alcohol (95%) | 10.0 wt% |
| Polyoxyethylene (20 moles) octyldodecanol | 1.0 |
| Pantothenyl ethyl ether | 0.1 |
| Linden extract | 1.5 |
| Methyl paraben | 0.15 |

| (B phase) | |
|---|---|
| Potassium hydroxide | 0.1 |

| (C phase) | |
|---|---|
| Glycerine | 5.0 |
| Dipropylene glycol | 10.0 |
| Carboxyvinyl polymer (product name: Carbopol 940 from B. F. Goodrich Chemical company) | 0.2 |
| Purified water | Balance |

### (Preparation method)

The A phase and C phase were, separately, each homogeneously dissolved. The A phase was then added to the C phase and solubilized. Finally, the B phase was added and a container was filled with the product.

### "Example 8 Facial pack"

| (Recipe) (A phase) | |
|---|---|
| Dipropylene glycol | 5.0 wt% |
| Polyoxyethylene (60 moles) hydrogenated castor oil | 5.0 |

| (B phase) | |
|---|---|
| Linden extract | 0.01 |
| Olive oil | 5.0 |
| Tocopherol acetate | 0.2 |
| Ethyl paraben | 0.2 |
| Perfume | 0.2 |

| (C phase) | |
|---|---|
| Polyvinyl alcohol (degree of saponification: 90, degree of polymerization: 2,000) | 13.0 |
| Ethanol | 7.0 |
| Purified water | Balance |

### (Preparation method)

Each of the A phase, B phase and C phase was homogeneously dissolved. The B phase was added to the A phase and solubilized. The C phase was then added and a container was filled with the product.

### "Example 9 Solid foundation"

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octanoate | 2.0 |
| Linden extract | 1.0 |
| Preservative | Appropriate amount |
| Perfume | Appropriate amount |

### (Preparation method)

The powder ingredients, from talc to black iron oxide as listed above, were thoroughly mixed with a blender. The oil ingredients, from squalane to isocetyl octanoate as listed above, as well as linden extract, the preservative and the perfume, were added and, after thorough kneading, a container was filled with the product.

### "Example 10 Emulsified foundation (cream type)"

| (Recipe) (Powder portion) | |
|---|---|
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene modified dimethyl polysiloxane | 4.0 |

| (Water phase) | |
|---|---|
| Purified water | 50.0 |
| 1,3-butylene glycol | 4.5 |
| Linden extract | 1.5 |
| Ascorbyl glucoside | 1.0 |
| Sorbitan sesquioleic ester | 3.0 |
| Preservative | Appropriate amount |
| Perfume | Appropriate amount |

### (Preparation method)

After heating and stirring the water phase, the powder portion was added to it and the mixture was treated with a homogenizer. The oil phase, heated and mixed, was then added and the resulting mixture was treated with a homogenizer. The perfume was then added while stirring and the product was cooled down to room temperature.

### [4] Examples for the inventions of claims 9-10

The immunopotentiating action and the effect of alleviating/preventing ultraviolet light-induced immunosuppression of clove extract were investigated by observing the prevention against the suppression of expression of the intercellular adhesive molecules-1 (ICAM-1) in Langerhans' cells due to UV irradiation.

### "Clove extract"

The clove extract used in the following examples was prepared by five-hour extraction of dried buds of clove (Syzygium aromaticum Merrill et Perry) in 50% ethanol at 50 °C, followed by filtering and removal of the solvent irom the filtrate to obtain a concentrate.

### [Testing methods and results: Prevention against the suppression of expression of the intercellular adhesive molecules-1 (ICAM-1) in Langerhans' cells due to UV irradiation.]

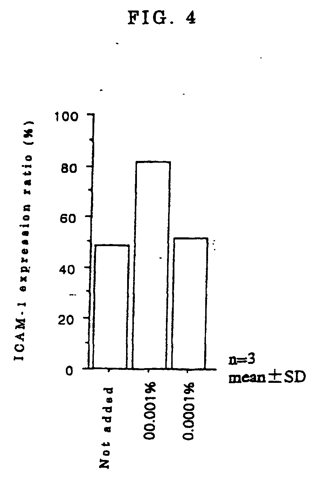

Langerhans' cells prepared by treating human skin epidermis with 0.5% trypsin was irradiated with UVA (5 J/cm², BLB lamp) and then cultured in a CO₂ incubator, with RPMI1640/10%FBS, for 24 hours at 37 °C . After the culture process, the cells were treated with the anti-MHC class II antibody labeled with FITC (from PharMingen) and the anti-ICAM-1 antibody labeled with PE (from PharMingen). A flow cytometer (XL from Epix) was used to analyse 3 x 10⁴ of the cells to measure the ICAM-1 expression. The result is shown in FIG. 4. The vertical axis of FIG. 4 shows the ICAM-1 expression ratio (%) and the horizontal axis shows the presence or absence of the clove extract (final concentration in wt% unit). FIG. 4 indicates that the suppression of expression of the intercellular adhesive molecules-1 (ICAM-1) in Langerhans' cells due to UV irradiation is prevented by the addition of the clove extract.

Examples of using clove extract as an immunopotentiator or a drug against immunosuppression are described below.

### "Example 1 Cream"

| (Recipe) | |
|---|---|
| Stearic acid | 5.0 wt% |
| Stearyl alcohol | 4.0 |
| Isopropylmyristate | 18.0 |
| Glycerine monostearic ester | 3.0 |
| Propylene glycol | 10.0 |
| Linden extract | 0.01 |
| Paraaminobenzoic acid | 0.5 |
| 2-ethylhexylparamethoxycinnamic acid | 5.0 |
| Caustic potash | 0.2 |
| Preservative | Appropriate amount |
| Perfume | Appropriate amount |
| Ion exchanged water | Balance |

### (Preparation method)

Propylene glycol, linden extract and caustic potash were added to ion exchanged water and dissolved, then heated up to and maintained at 70 °C (water phase). The other ingredients were mixed and heat-melted and then the temperature was maintained at 70 °C (oil phase). The oil phase was gradually added to the water phase and, after all was added, the temperature was maintained at the same temperature to allow the reaction to occur. Finally, the product was homogeneously emulsified using a homogenizer and cooled to 30 °C while being thoroughly stirred.

### "Example 2 Cream"

| (Recipe) | |
|---|---|
| Stearic acid | 2.0 wt% |
| Stearyl alcohol | 7.0 |
| Lanolin hydrate | 2.0 |
| Squalane | 5.0 |
| 2-octyldodecyl alcohol | 6.0 |
| Polyoxyethylene (25 moles) cetyl alcohol ether | 3.0 |
| Glycerine monostearic ester | 2.0 |
| Propylene glycol | 5.0 |
| 2-ethylhexylparamethoxycinnamic acid | 10.0 |
| Linden extract | 0.05 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and, after pre-emulsification, the product was homogeneously emulsified using a homogenizer and cooled to 30 °C while being thoroughly stirred.

### "Example 3 Cream"

| (Recipe) | |
|---|---|
| Solid paraffin | 5.0 wt% |
| Beeswax | 10.0 |
| Vaseline | 15.0 |
| Liquid paraffin | 41.0 |
| Glycerine monostearic ester | 2.0 |
| Polyoxyethylene (20 moles) sorbitan monolauric ester | 2.0 |
| Soap powder | 0.1 |
| 2-ethylhexylparamethoxycinnamic acid | 1.0 |
| Borax | 0.2 |
| Linden extract | 0.05 |
| Ascorbic acid | 2.0 |
| Ethyl paraben | 0.3 |
| Perfume | Appropriate amount |
| Ion exchanged water | Balance |

### (Preparation method)

Soap powder and borax were added to ion exchanged water and dissolved, heated up to and maintained at 70 °C (water phase). The other ingredients were mixed and heat-melted and then the temperature was maintained at 70 °C (oil phase). The oil phase was gradually added to the water phase while being stirred to initiate the reaction. After the completion of the reaction, the product was homogeneously emulsified using a homogenizer and cooled to 30 °C while being thoroughly stirred.

### "Example 4 Emulsion"

| (Recipe) | |
|---|---|
| Stearic acid | 2.5 wt% |
| Cetyl alcohol | 1.5 |
| Vaseline | 5.0 |
| Liquid paraffin | 10.0 |
| Polyoxyethylene (10 moles) monooleic ester | 2.0 |
| Polyethylene glycol 1500 | 3.0 |
| Triethanolamine | 1.0 |
| Carboxyvinyl polymer (product name: Carbopol 941 from B. F. Goodrich Chemical company) | 0.05 |
| Linden extract | 0.01 |
| Octyl paradimethylaminobenzoate | 1.0 |
| Arbutin | 3.5 |
| Ethyl paraben | 0.3 |
| Perfume | Appropriate amount |
| Ion exchanged water | Balance |

### (Preparation method)

The carboxyvinyl polymer was dissolved in a small amount of ion exhanged water (A phase). Polyethylene glycol 1500 and triethanolamine were added to the rest of the ion exchanged water and heated and dissolved, after which the temperature was maintained at 70 °C (water phase). The other ingredients were mixed and heat-melted and then the temperature was maintained at 70 °C (oil phase). The oil phase was added to the water phase and, after pre-emulsification, the A phase was added. The product was then homogeneously emulsified using a homogenizer and cooled to 30 °C while being thoroughly stirred.

### "Example 5 Emulsion"

| (Recipe) | |
|---|---|
| Microcrystalline wax | 1.0 wt% |
| Glutathione | 1.0 |
| Beeswax | 2.0 |
| Lanolin | 20.0 |
| Liquid paraffin | 10.0 |
| Octyl paramethylaminobenzoate | 3.0 |
| 2-ethylhexylparamethoxycinnamic acid | 5.0 |
| Squalane | 5.0 |
| Sorbitan sesquioleic ester | 4.0 |
| Polyoxyethylene (20 moles) sorbitan monooleic ester | 1.0 |
| Propylene glycol | 7.0 |
| Linden extract | 10.0 |
| Magnesium ascrobate phosphate | 3.0 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and homogeneously emulsified using a homogenizer. After emulsification, the product was cooled to 30 °C while being thoroughly stirred.

### "Example 6 Jelly"

| (Recipe) | |
|---|---|
| 95% ethyl alcohol | 10.0 wt% |
| Dipropylene glycol | 15.0 |
| Polyoxyethylene (50 moles) oleyl alcohol ether | 2.0 |
| Carboxyvinyl polymer (product name: Carbopol 940 from B. F. Goodrich Chemical company) | 1.0 |
| Caustic soda | 0.15 |
| L-arginine | 0.1 |
| Isopropyl paramethoxycinnamate | 0.1 |
| 2-ethylhexylparamethoxycinnamic acid | 0.5 |
| Titanium oxide | 5.0 |
| Linden extract | 7.0 |
| Sodium 2-hydroxy-4-methoxybenzophenonesulfonate | 0.05 |
| Ethylenediamine-tetraacetic acid trisodium dihydrate | 0.05 |
| Methyl paraben | 0.2 |
| Perfume | Appropriate amount |
| Ion exchanged water | Balance |

### (Preparation method)

Carbopol 940 was homogeneously dissolved in ion exhanged water. Linden extract and polyoxyethylene (50 moles) oleyl alcohol ether were dissolved in 95% ethanol and added to the water phase. The other ingredients were added and the mixture was neutralized and thickened by caustic soda and/or L-arginine.

### "Example 7 Essence"

| (Recipe) (A phase) | |
|---|---|
| Ethyl alcohol (95%) | 10.0 wt% |
| Polyoxyethylene (20 moles) octyldodecanol | 1.0 |
| Pantothenyl ethyl ether | 0.1 |
| Linden extract | 1.5 |
| Methyl paraben | 0.15 |

| (B phase) | |
|---|---|
| Potassium hydroxide | 0.1 |

| (C phase) | |
|---|---|
| Glycerine | 5.0 |
| Dipropylene glycol | 10.0 |
| Carboxyvinyl polymer (product name: Carbopol 940 from B. F. Goodrich Chemical company) | 0.2 |
| Purified water | Balance |

### (Preparation method)

The A phase and C phase were, separately, each homogeneously dissolved. The A phase was then added to the C phase and solubilized. Finally, the B phase was added and a container was filled with the product.

### "Example 8 Facial pack"

| (Recipe) (A phase) | |
|---|---|
| Dipropylene glycol | 5.0 wt% |
| Polyoxyethylene (60 moles) hydrogenated castor oil | 5.0 |

| (B phase) | |
|---|---|
| Linden extract | 0.01 |
| Olive oil | 5.0 |
| Tocopherol acetate | 0.2 |
| Ethyl paraben | 0.2 |
| Perfume | 0.2 |

| (C phase) | |
|---|---|
| Polyvinyl alcohol (degree of saponification: 90, degree of polymerization: 2,000) | 13.0 |
| Ethanol | 7.0 |
| Purified water | Balance |

### (Preparation method)

Each of the A phase, B phase and C phase was homogeneously dissolved. The B phase was added to the A phase and solubilized. The C phase was then added and a container was filled with the product.

### "Example 9 Solid foundation"

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octanoate | 2.0 |
| Linden extract | 1.0 |
| Preservative | Appropriate amount |
| Perfume | Appropriate amount |

### (Preparation method)

The powder ingredients, from talc to black iron oxide as listed above, were thoroughly mixed with a blender. The oil ingredients, from squalane to isocetyl octanoate as listed above, as well as linden extract, the preservative and the perfume, were added and, after thorough kneading, a container was filled with the product.

### "Example 10 Emulsified foundation (cream type)"

| (Recipe) (Powder portion) | |
|---|---|
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene modified dimethyl polysiloxane | 4.0 |

| (Water phase) | |
|---|---|
| Purified water | 50.0 |
| 1,3-butylene glycol | 4.5 |
| Linden extract | 1.5 |
| Ascorbyl glucoside | 1.0 |
| Sorbitan sesquioleic ester | 3.0 |
| Preservative | Appropriate amount |
| Perfume | Appropriate amount |

### (Preparation method)

After heating and stirring the water phase, the powder portion was added to it and the mixture was treated with a homogenizer. The oil phase, heated and mixed, was then added and the resulting mixture was treated with a homogenizer. The perfume was then added while stirring and the product was cooled down to room temperature.

### [5] Examples for the inventions of claims 11-12

The immunopotentiating action and the effect of alleviating/preventing ultraviolet light-induced immunosuppression of Geranium herb extract were investigated by observing the prevention against the suppression of expression of the intercellular adhesive molecules-1 (ICAM-1) in Langerhans' cells due to UV irradiation.

### "Geranium herb extract"

The Geranium herb extract used in the following examples was prepared by five-hour extraction of the thinly sliced above-ground part of Geranium thunbergii in 50% ethanol at 50 °C , followed by filtering and removal of the solvent irom the filtrate to obtain a concentrate.

### [Testing methods and results: Prevention against the suppression of expression of the intercellular adhesive molecules-1 (ICAM-1) in Langerhans' cells due to UV irradiation.]

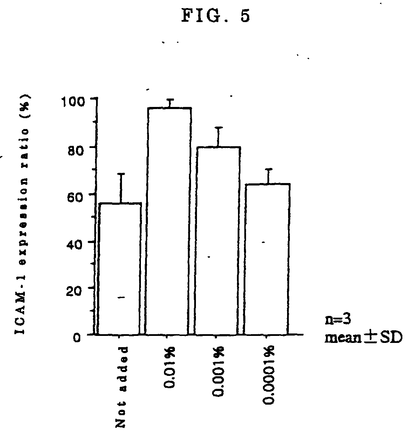

Langerhans' cells prepared by treating human skin epidermis with 0.5% trypsin was irradiated with UVA (5 J/cm², BLB lamp) and then cultured in a CO₂ incubator, with RPMI1640/10%FBS, for 24 hours at 37 °C . After the culture process, the cells were treated with the anti-MHC class II antibody labeled with FITC (from PharMingen) and the anti-ICAM-1 antibody labeled with PE (from PharMingen). A flow cytometer (XL from Epix) was used to analyse 3 x 10⁴ of the cells to measure the ICAM-1 expression. The result is shown in FIG. 5. The vertical axis of FIG. 5 shows the ICAM-1 expression ratio (%) and the horizontal axis shows the presence or absence of the Geranium herb extract (final concentration in wt% unit). FIG. 5 indicates that the suppression of expression of the intercellular adhesive molecules-1 (ICAM-1) in Langerhans' cells due to UV irradiation is prevented by the addition of the Geranium herb extract.

Examples of using Geranium herb extract as an immunopotentiator or a drug against immunosuppression are described below.

### "Example 1 Cream"

| (Recipe) | |
|---|---|
| Stearic acid | 5.0 wt% |
| Stearyl alcohol | 4.0 |
| Isopropylmyristate | 18.0 |
| Glycerine monostearic ester | 3.0 |
| Propylene glycol | 10.0 |
| Geranium herb extract | 0.01 |
| Paraaminobenzoic acid | 0.5 |
| 2-ethylhexylparamethoxycinnamic acid | 5.0 |
| Caustic potash | 0.2 |
| Preservative | Appropriate amount |
| Perfume | Appropriate amount |
| Ion exchanged water | Balance |

### (Preparation method)

Propylene glycol, Geranium herb extract and caustic potash were added to ion exchanged water and dissolved, then heated up to and maintained at 70 °C (water phase). The other ingredients were mixed and heat-melted and then the temperature was maintained at 70 °C (oil phase). The oil phase was gradually added to the water phase and, after all was added, the temperature was maintained at the same temperature to allow the reaction to occur. Finally, the product was homogeneously emulsified using a homogenizer and cooled to 30 °C while being thoroughly stirred.

### "Example 2 Cream"

| (Recipe) | |
|---|---|
| Stearic acid | 2.0 wt% |
| Stearyl alcohol | 7.0 |
| Lanolin hydrate | 2.0 |
| Squalane | 5.0 |
| 2-octyldodecyl alcohol | 6.0 |
| Polyoxyethylene (25 moles) cetyl alcohol ether | 3.0 |
| Glycerine monostearic ester | 2.0 |
| Propylene glycol | 5.0 |
| 2-ethylhexylparamethoxycinnamic acid | 10.0 |
| Geranium herb extract | 0.05 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and, after pre-emulsification, the product was homogeneously emulsified using a homogenizer and cooled to 30 °C while being thoroughly stirred.

### "Example 3 Cream"

| (Recipe) | |
|---|---|
| Solid paraffin | 5.0 wt% |
| Beeswax | 10.0 |
| Vaseline | 15.0 |
| Liquid paraffin | 41.0 |
| Glycerine monostearic ester | 2.0 |
| Polyoxyethylene (20 moles) sorbitan monolauric ester | 2.0 |
| Soap powder | 0.1 |
| 2-ethylhexylparamethoxycinnamic acid | 1.0 |
| Borax | 0.2 |
| Geranium herb extract | 0.05 |
| Ascorbic acid | 2.0 |
| Ethyl paraben | 0.3 |
| Perfume | Appropriate amount |
| Ion exchanged water | Balance |

### (Preparation method)

Soap powder and borax were added to ion exchanged water and dissolved, heated up to and maintained at 70 °C (water phase). The other ingredients were mixed and heat-melted and then the temperature was maintained' at 70 °C (oil phase). The oil phase was gradually added to the water phase while being stirred to initiate the reaction. After the completion of the reaction, the product was homogeneously emulsified using a homogenizer and cooled to 30 °C while being thoroughly stirred.

### "Example 4 Emulsion"

| (Recipe) | |
|---|---|
| Stearic acid | 2.5 wt% |
| Cetyl alcohol | 1.5 |
| Vaseline | 5.0 |
| Liquid paraffin | 10.0 |
| Polyoxyethylene (10 moles) monooleic ester | 2.0 |
| Polyethylene glycol 1500 | 3.0 |
| Triethanolamine | 1.0 |
| Carboxyvinyl polymer (product name: Carbopol 941 from B. F. Goodrich Chemical company) | 0.05 |
| Geranium herb extract | 0.01 |
| Octyl paradimethylaminobenzoate | 1.0 |
| Sodium hydrogensulfite | 0.01 |
| Arbutin | 3.5 |
| Ethyl paraben | 0.3 |
| Perfume | Appropriate amount |
| Ion exchanged water | Balance |

### (Preparation method)

The carboxyvinyl polymer was dissolved in a small amount of ion exhanged water (A phase). Polyethylene glycol 1500 and triethanolamine were added to the rest of the ion exchanged water and heated and dissolved, after which the temperature was maintained at 70 °C (water phase). The other ingredients were mixed and heat-melted and then the temperature was maintained at 70 °C (oil phase). The oil phase was added to the water phase and, after pre-emulsification, the A phase was added. The product was then homogeneously emulsified using a homogenizer and cooled to 30 °C while being thoroughly stirred.

### "Example 5 Emulsion"

| (Recipe) | |
|---|---|
| Microcrystalline wax | 1.0 wt% |
| Glutathione | 1.0 |
| Beeswax | 2.0 |
| Lanolin | 20.0 |
| Liquid paraffin | 10.0 |
| Octyl paramethylaminobenzoate | 3.0 |
| 2-ethylhexylparamethoxycinnamic acid | 5.0 |
| Squalane | 5.0 |
| Sorbitan sesquioleic ester | 4.0 |
| Polyoxyethylene (20 moles) sorbitan monooleic ester | 1.0 |
| Propylene glycol | 7.0 |
| Geranium herb extract | 10.0 |
| Magnesium ascrobate phosphate | 3.0 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and homogeneously emulsified using a homogenizer. After emulsification, the product was cooled to 30 °C while being thoroughly stirred.

### "Example 6 Jelly"

| (Recipe) | |
|---|---|
| 95% ethyl alcohol | 10.0 wt% |
| Dipropylene glycol | 15.0 |
| Polyoxyethylene (50 moles) oleyl alcohol ether | 2.0 |
| Carboxyvinyl polymer (product name: Carbopol 940 from B. F. Goodrich Chemical company) | 1.0 |
| Caustic soda | 0.15 |
| L-arginine | 0.1 |
| Isopropyl paramethoxycinnamate | 0.1 |
| 2-ethylhexylparamethoxycinnamic acid | 0.5 |
| Titanium oxide | 5.0 |
| Geranium herb extract | 7.0 |
| Sodium 2-hydroxy-4-methoxybenzophenonesulfonate | 0.05 |
| Ethylenediamine-tetraacetic acid trisodium dihydrate | 0.05 |
| Methyl paraben | 0.2 |
| Perfume | Appropriate amount |
| Ion exchanged water | Balance |

### (Preparation method)

Carbopol 940 was homogeneously dissolved in ion exhanged water. Geranium herb extract and polyoxyethylene (50 moles) oleyl alcohol ether were dissolved in 95% ethanol and added to the water phase. The other ingredients were added and the mixture was neutralized and thickened by caustic soda and/or L-arginine.

### "Example 7 Essence"

| (Recipe) (A phase) | |
|---|---|
| Ethyl alcohol (95%) | 10.0 wt% |
| Polyoxyethylene (20 moles) octyldodecanol | 1.0 |
| Pantothenyl ethyl ether | 0.1 |
| Geranium herb extract | 1.5 |
| Methyl paraben | 0.15 |

| (B phase) | |
|---|---|
| Potassium hydroxide | 0.1 |

| (C phase) | |
|---|---|
| Glycerine | 5.0 |
| Dipropylene glycol | 10.0 |
| Carboxyvinyl polymer (product name: Carbopol 940 from B. F. Goodrich Chemical company) | 0.2 |
| Purified water | Balance |

### (Preparation method)

The A phase and C phase were, separately, each homogeneously dissolved. The A phase was then added to the C phase and solubilized. Finally, the B phase was added and a container was filled with the product.

### "Example 8 Facial pack"

| (Recipe) (A phase) | |
|---|---|
| Dipropylene glycol | 5.0 wt% |
| Polyoxyethylene (60 moles) hydrogenated castor oil | 5.0 |

| (B phase) | |
|---|---|
| Geranium herb extract | 0.01 |
| Olive oil | 5.0 |
| Tocopherol acetate | 0.2 |
| Ethyl paraben | 0.2 |
| Perfume | 0.2 |

| (C phase) | |
|---|---|
| Polyvinyl alcohol (degree of saponification: 90, degree of polymerization: 2,000) | 13.0 |
| Ethanol | 7.0 |
| Purified water | Balance |

### (Preparation method)

Each of the A phase, B phase and C phase was homogeneously dissolved. The B phase was added to the A phase and solubilized. The C phase was then added and a container was filled with the product.

### "Example 9 Solid foundation"

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octanoate | 2.0 |
| Geranium herb extract | 1.0 |
| Preservative | Appropriate amount |
| Perfume | Appropriate amount |

### (Preparation method)

The powder ingredients, from talc to black iron oxide as listed above, were thoroughly mixed with a blender. The oil ingredients, from squalane to isocetyl octanoate as listed above, as well as Geranium herb extract, the preservative and the perfume, were added and, after thorough kneading, a container was filled with the product.

### "Example 10 Emulsified foundation (cream type)"

| (Recipe) (Powder portion) | |
|---|---|
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene modified dimethyl polysiloxane | 4.0 |

| (Water phase) | |
|---|---|
| Purified water | 50.0 |
| 1,3-butylene glycol | 4.5 |
| Geranium herb extract | 1.5 |
| Ascorbyl glucoside | 1.0 |
| Sorbitan sesquioleic ester | 3.0 |
| Preservative | Appropriate amount |
| Perfume | Appropriate amount |

### (Preparation method)

After heating and stirring the water phase, the powder portion was added to it and the mixture was treated with a homogenizer. The oil phase, heated and mixed, was then added and the resulting mixture was treated with a homogenizer. The perfume was then added while stirring and the product was cooled down to room temperature.

### [6] Examples for the inventions of claims 13-14

The immunopotentiating action and the effect of alleviating/preventing ultraviolet light-induced immunosuppression of rosemary extract were investigated by observing the prevention against the suppression of expression of the intercellular adhesive molecules-1 (ICAM-1) in Langerhans' cells due to UV irradiation.

### "Rosemary extract"

The rosemary extract used in the following examples was prepared by five-hour extraction of the thinly sliced flowers of roesmary in 50% ethanol at 50 °C, followed by filtering and removal of the solvent irom the filtrate to obtain a concentrate.

### [Testing methods and results: Prevention against the suppression of expression of the intercellular adhesive molecules-1 (ICAM-1) in Langerhans' cells due to UV irradiation.]

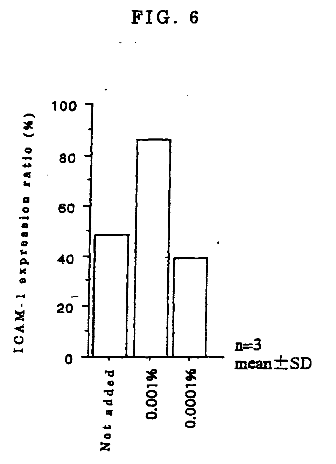

Langerhans' cells prepared by treating human skin epidermis with 0.5% trypsin was irradiated with UVA (5 J/cm², BLB lamp) and then cultured in a CO₂ incubator, with RPMI1640/10%FBS, for 24 hours at 37 °C . After the culture process, the cells were treated with the anti-MHC class II antibody labeled with FITC (from PharMingen) and the anti-ICAM-1 antibody labeled with PE (from PharMingen). A flow cytometer (XL from Epix) was used to analyse 3 x 10⁴ of the cells to measure the ICAM-1 expression. The result is shown in FIG. 6 The vertical axis of FIG. 6 shows the ICAM-1 expression ratio (%) and the horizontal axis shows the presence or absence of the rosemary extract (final concentration in wt% unit). FIG. 6 indicates that the suppression of expression of the intercellular adhesive molecules-1 (ICAM-1) in Langerhans' cells due to UV irradiation is prevented by the addition of the rosemary extract.

Examples of using rosemary extract as an immunopotentiator or a drug against immunosuppression are described below.

### "Example 1 Cream"

| (Recipe) | |
|---|---|
| Stearic acid | 5.0 wt% |
| Stearyl alcohol | 4.0 |
| Isopropylmyristate | 18.0 |
| Glycerine monostearic ester | 3.0 |
| Propylene glycol | 10.0 |
| Rosemary extract | 0.01 |
| Paraaminobenzoic acid | 0.5 |
| 2-ethylhexylparamethoxycinnamic acid | 5.0 |
| Caustic potash | 0.2 |
| Preservative | Appropriate amount |
| Perfume | Appropriate amount |
| Ion exchanged water | Balance |

### (Preparation method)

Propylene glycol, rosemary extract and caustic potash were added to ion exchanged water and dissolved, then heated up to and maintained at 70 °C (water phase). The other ingredients were mixed and heat-melted and then the temperature was maintained at 70 °C (oil phase). The oil phase was gradually added to the water phase and, after all was added, the temperature was maintained at the same temperature to allow the reaction to occur. Finally, the product was homogeneously emulsified using a homogenizer and cooled to 30 °C while being thoroughly stirred.

### "Example 2 Cream"

| (Recipe) | |
|---|---|
| Stearic acid | 2.0 wt% |
| Stearyl alcohol | 7.0 |
| Lanolin hydrate | 2.0 |
| Squalane | 5.0 |
| 2-octyldodecyl alcohol | 6.0 |
| Polyoxyethylene (25 moles) cetyl alcohol ether | 3.0 |
| Glycerine monostearic ester | 2.0 |
| Propylene glycol | 5.0 |
| 2-ethylhexylparamethoxycinnamic acid | 10.0 |
| Rosemary extract | 0.05 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and, after pre-emulsification, the product was homogeneously emulsified using a homogenizer and cooled to 30 °C while being thoroughly stirred.

### "Example 3 Cream"

| (Recipe) | |
|---|---|
| Solid paraffin | 5.0 wt% |
| Beeswax | 10.0 |
| Vaseline | 15.0 |
| Liquid paraffin | 41.0 |
| Glycerine monostearic ester | 2.0 |
| Polyoxyethylene (20 moles) sorbitan monolauric ester | 2.0 |
| Soap powder | 0.1 |
| 2-ethylhexylparamethoxycinnamic acid | 1.0 |
| Borax | 0.2 |
| Rosemary extract | 0.05 |
| Ascorbic acid | 2.0 |
| Ethyl paraben | 0.3 |
| Perfume | Appropriate amount |
| Ion exchanged water | Balance |

### (Preparation method)

Soap powder and borax were added to ion exchanged water and dissolved, heated up to and maintained at 70 °C (water phase). The other ingredients were mixed and heat-melted and then the temperature was maintained at 70 °C (oil phase). The oil phase was gradually added to the water phase while being stirred to initiate the reaction. After the completion of the reaction, the product was homogeneously emulsified using a homogenizer and cooled to 30 °C while being thoroughly stirred.

### "Example 4 Emulsion"

| (Recipe) | |
|---|---|
| Stearic acid | 2.5 wt% |
| Cetyl alcohol | 1.5 |
| Vaseline | 5.0 |
| Liquid paraffin | 10.0 |
| Polyoxyethylene (10 moles) monooleic ester | 2.0 |
| Polyethylene glycol 1500 | 3.0 |
| Triethanolamine | 1.0 |
| Carboxyvinyl polymer (product name: Carbopol 941 from B. F. Goodrich Chemical company) | 0.05 |
| Rosemary extract | 0.01 |
| Octyl paradimethylaminobenzoate | 1.0 |
| Sodium hydrogensulfite | 0.01 |
| Arbutin | 3.5 |
| Ethyl paraben | 0.3 |
| Perfume | Appropriate amount |
| Ion exchanged water | Balance |

### (Preparation method)

The carboxyvinyl polymer was dissolved in a small amount of ion exhanged water (A phase). Polyethylene glycol 1500 and triethanolamine were added to the rest of the ion exchanged water and heated and dissolved, after which the temperature was maintained at 70 °C (water phase). The other ingredients were mixed and heat-melted and then the temperature was maintained at 70 °C (oil phase). The oil phase was added to the water phase and, after pre-emulsification, the A phase was added. The product was then homogeneously emulsified using a homogenizer and cooled to 30 °C while being thoroughly stirred.

### "Example 5 Emulsion"

| (Recipe) | |
|---|---|
| Microcrystalline wax | 1.0 wt% |
| Glutathione | 1.0 |
| Beeswax | 2.0 |
| Lanolin | 20.0 |
| Liquid paraffin | 10.0 |
| Octyl paramethylaminobenzoate | 3.0 |
| 2-ethylhexylparamethoxycinnamic acid | 5.0 |
| Squalane | 5.0 |
| Sorbitan sesquioleic ester | 4.0 |
| Polyoxyethylene (20 moles) sorbitan monooleic ester | 1.0 |
| Propylene glycol | 7.0 |
| Rosemary extract | 10.0 |
| Magnesium ascrobate phosphate | 3.0 |
| Ethyl paraben | 0.3 |
| Perfume | Appropriate amount |
| Ion exchanged water | Balance |

### (Preparation method)

Propylene glycol was added to ion exchanged water and heated up to and maintained at 70 °C (water phase). The other ingredients were mixed and heat-melted and then the temperature was maintained at 70 °C (oil phase). The oil phase was added to the water phase and homogeneously emulsified using a homogenizer. After emulsification, the product was cooled to 30 °C while being thoroughly stirred.

### "Example 6 Jelly"

| (Recipe) | |
|---|---|
| 95% ethyl alcohol | 10.0 wt% |
| Dipropylene glycol | 15.0 |
| Polyoxyethylene (50 moles) oleyl alcohol ether | 2.0 |
| Carboxyvinyl polymer (product name: Carbopol 940 from B. F. Goodrich Chemical company) | 1.0 |
| Caustic soda | 0.15 |
| L-arginine | 0.1 |
| Isopropyl paramethoxycinnamate | 0.1 |
| 2-ethylhexylparamethoxycinnamic acid | 0.5 |
| Titanium oxide | 5.0 |
| Rosemary extract | 7.0 |
| Sodium 2-hydroxy-4-methoxybenzophenonesulfonate | 0.05 |
| Ethylenediamine-tetraacetic acid trisodium dihydrate | 0.05 |
| Methyl paraben | 0.2 |
| Perfume | Appropriate amount |
| Ion exchanged water | Balance |

### (Preparation method)

Carbopol 940 was homogeneously dissolved in ion exhanged water. Rosemary extract and polyoxyethylene (50 moles) oleyl alcohol ether were dissolved in 95% ethanol and added to the water phase. The other ingredients were added and the mixture was neutralized and thickened by caustic soda and/or L-arginine.

### "Example 7 Essence"

| (Recipe) (A phase) | |
|---|---|
| Ethyl alcohol (95%) | 10.0 wt% |
| Polyoxyethylene (20 moles) octyldodecanol | 1.0 |
| Pantothenyl ethyl ether | 0.1 |
| Rosemary extract | 1.5 |
| Methyl paraben | 0.15 |

| (B phase) | |
|---|---|
| Potassium hydroxide | 0.1 |

| (C phase) | |
|---|---|
| Glycerine | 5.0 |
| Dipropylene glycol | 10.0 |
| Carboxyvinyl polymer (product name: Carbopol 940 from B. F. Goodrich Chemical company) | 0.2 |
| Purified water | Balance |

### (Preparation method)

The A phase and C phase were, separately, each homogeneously dissolved. The A phase was then added to the C phase and solubilized. Finally, the B phase was added and a container was filled with the product.

### "Example 8 Facial pack"

| (Recipe) (A phase) | |
|---|---|
| Dipropylene glycol | 5.0 wt% |
| Polyoxyethylene (60 moles) hydrogenated castor oil | 5.0 |

| (B phase) | |
|---|---|
| Rosemary extract | 0.01 |
| Olive oil | 5.0 |
| Tocopherol acetate | 0.2 |
| Ethyl paraben | 0.2 |
| Perfume | 0.2 |

| (C phase) | |
|---|---|
| Polyvinyl alcohol (degree of saponification: 90, degree of polymerization: 2,000) | 13.0 |
| Ethanol | 7.0 |
| Purified water | Balance |

### (Preparation method)

Each of the A phase, B phase and C phase was homogeneously dissolved. The B phase was added to the A phase and solubilized. The C phase was then added and a container was filled with the product.

### "Example 9 Solid foundation"

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octanoate | 2.0 |
| Rosemary extract | 1.0 |
| Preservative | Appropriate amount |
| Perfume | Appropriate amount |

### (Preparation method)

The powder ingredients, from talc to black iron oxide as listed above, were thoroughly mixed with a blender. The oil ingredients, from squalane to isocetyl octanoate as listed above, as well as rosemary extract, the preservative and the perfume, were added and, after thorough kneading, a container was filled with the product.

### "Example 10 Emulsified foundation (cream type)"

| (Recipe) (Powder portion) | |
|---|---|
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene modified dimethyl polysiloxane | 4.0 |

| (Water phase) | |
|---|---|
| Purified water | 50.0 |
| 1,3-butylene glycol | 4.5 |
| Rosemary extract | 1.5 |
| Ascorbyl glucoside | 1.0 |
| Sorbitan sesquioleic eater | 3.0 |
| Preservative | Appropriate amount |
| Perfume | Appropriate amount |

### (Preparation method)

After heating and stirring the water phase, the powder portion was added to it and the mixture was treated with a homogenizer. The oil phase, heated and mixed, was then added and the resulting mixture was treated with a homogenizer. The perfume was then added while stirring and the product was cooled down to room temperature.

### INDUSTRIAL APPLICABILITY OF THE INVENTION

The present invention can provide a superior skin immunopotentiator or drug against skin immunosuppression which, through external application, prevents a reduction in the skin immune functions due to ultraviolet light.

## Claims

1. An immunopotentiator for preventing ultraviolet light-induced skin immunosuppression which characteristically contains glutathione.

2. A drug against ultraviolet light-induced skin immunosuppression which characteristically contains glutathione.

3. An immunopotentiating endermic liniment for preventing ultraviolet light-induced skin immunosuppression.

4. An endermic liniment against ultraviolet light-induced skin immunosuppression which characteristically contains glutathione.

5. An immunopotentiator for preventing ultraviolet light-induced skin immunosuppression which characteristically contains Scutellaria root extract.

6. A drug against ultraviolet light-induced skin immunosuppression which characteristically contains Scutellaria root extract.

7. An immunopotentiator which characteristically contains linden extract.

8. A drug against immunosuppression which characteristically contains linden extract.

9. An immunopotentiator which characteristically contains clove extract.

10. A drug against immunosuppression which characteristically contains clove extract.

11. An immunopotentiator which characteristically contains Geranium herb extract.

12. A drug against immunosuppression which characteristically contains Geranium herb extract.

13. An immunopotentiator which characteristically contains rosemary extract.

14. A drug against immunosuppression which characteristically contains rosemary extract.
